**Europäisches Patentamt**

**European Patent Office**

**Office européen des brevets**

(19)

(11) Veröffentlichungsnummer: **0 115 313**
**A2**

(12) # EUROPÄISCHE PATENTANMELDUNG

(21) Anmeldenummer: **84100651.3**

(22) Anmeldetag: **23.01.84**

(51) Int. Cl.³: **B 01 F 17/22**

(30) Priorität: **31.01.83 DE 3303174**

(43) Veröffentlichungstag der Anmeldung:
**08.08.84** Patentblatt **84/32**

(84) Benannte Vertragsstaaten:
**AT BE CH DE FR GB IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Borggrefe, Gerhard, Dr.**
**Platanenstrasse 19**
**D-4000 Düsseldorf(DE)**

(72) Erfinder: **Hase, Christian, Dr.**
**Millrather Weg 29**
**D-4006 Erkrath(DE)**

(54) **Stabile Öl-in-Wasser-Emulsionen mit hohem Ölgehalt.**

(57) Stabile Öl-in-Wasser-Emulsionen mit einem Gehalt an nichtwäßriger Phase von mehr als 60 Gew.-% lassen sich herstellen unter Verwendung von wasserlöslichen Salzen von Monoacylcyanamiden, deren Acylrest 10 - 20 C-Atome aufweist, als Emulgatoren. Bevorzugt werden die Monoacylcyanamide von Fettsäuren mit 12 - 18 C-Atomen in Form der Natriumsalze eingesetzt. Die Öl-in-Wasser-Emulsionen enthalten bevorzugt 70 - 95 Gew.-% nichtwäßrige Phase und 0,1 - 3 Gew.-% an Emulgator.

EP 0 115 313 A2

4000 Düsseldorf, den 27.01.1983
Henkelstraße 67

**0115313**
**HENKEL KGaA**
ZR-FE/Patente

Dr. Wa/Rk

P a t e n t a n m e l d u n g

D 6350 EP

"Stabile Öl-in-Wasser-Emulsion mit hohem Ölgehalt"

---

Aus der DE-A1-16 44 942 sind stabile, thixotrope Öl-in-Wasser-Emulsionen bekannt, deren Gehalt an nichtwäßriger innerer Phase mindestens 80 Vol.-%, vorzugsweise bis zu 95 Vol.-%, beträgt. Die zur Herstellung dieser Emulsionen verwendeten Emulgatoren bestehen in erster Linie aus Alkoxylierungsprodukten von höhermolekularen Alkoholen, Alkoxyphenolen, Carbonsäuren, Aminen, Amiden oder Polyolen.

Die Herstellung dieser Emulsionen ist jedoch verhältnismäßig umständlich, da zu Beginn der Emulsionsbildung die Zugabe des zu emulgierenden Öls zu der wäßrigen, emulgatorhaltigen Phase in kleinen Schritten und über einen längeren Zeitraum verteilt erfolgen muß. Die zur Bildung stabiler Emulsionen notwendige volle Wirkung entfaltet der Emulgator erst in einem fortgeschrittenen Stadium der Emulsionsbildung. Im technischen Bereich führt eine solche Arbeitsweise zu einem größeren Zeitbedarf und erhöhtem technischen und personellen Aufwand für die Bedienungs- und Überwachungsaufgaben.

Es ergab sich daraus die Aufgabe, ein Emulgatorsystem zu entwickeln, dessen Wirkung für eine vollständige Emulsionsbildung auch bei einmaliger Zugabe der Gesamtmenge an nichtwäßriger Phase zur wäßrigen, emulgatorhaltigen Phase ausreicht.

Gegenstand der Erfindung sind stabile Öl-in-Wasser-Emulsionen mit einem Gehalt an nichtwäßriger Phase von mehr als 60 Gew.-%,, dadurch gekennzeichnet, daß der Emulgator aus wasserlöslichen Salzen von Monoacylcyanamiden mit 10 - 20 Kohlenstoffatomen in der Acylgruppe besteht.

...

0115313
HENKEL KGaA
ZR-FE/Patente

Monoacylcyanamide sowie deren Salze sind aus der DE-PS 706 428 und der GB-PS 428 091 bekannt. Sie sind durch Umsetzung von Fettsäuren bzw. Fettsäurederivaten mit Cyanamid oder Alkalicyanamid erhältlich. Soweit hierbei saure Monoacylcyanamide entstehen, werden sie durch Neutralisation mit Alkalien oder Ammoniumbasen in die entsprechenden neutralen Salze überführt.

In den zitierten Patentschriften ist die Verwendung der Verbindungen als Seifenersatz sowie als Netz- und Dispergiermittel genannt. Eine Lehre des Inhaltes, daß die Salze der Monoacylcyanamide sich zur Herstellung von Öl-in-Wasser-Emulsionen mit hohem, d.h. 60 Gew.-% übersteigenden Ölgehalt eignen, ist dem Stand der Technik nicht zu entnehmen.

Die für eine schnelle und vollständige Bildung stabiler Emulsionen erforderliche Menge an Emulgator ist vergleichsweise gering und beträgt vorzugsweise 0,1 - 3 Gew.-% und insbesondere 0,5 - 2 Gew.-%, bezogen auf die Emulsion. Die Acylcyanamide liegen in Form der wasserlöslichen Salze, z.B. als Salz des Natriums, Kaliums, Magnesiums, Ammoniums sowie von Ammoniumbasen, wie des Mono-, Di- oder Triethanolamins vor. Vorzugsweise kommen sie als Natriumsalz zum Einsatz. Die Acylreste können aliphatischer, cycloaliphatischer oder alkylaromatischer Natur sein. Vorzugsweise leiten sie sich von Fettsäuren mit 12 - 18 Kohlenstoffatomen ab, wobei diese Fettsäuren gesättigt oder ungesättigt sein können und vorzugsweise geradkettig sind.

Geeignete Fettsäuren bzw. Fettsäuregemische können aus Fetten natürlichen Ursprungs, wie Kokosfett, Talg, Palmkernöl, Palmöl, Tallöl, Baumwollsaatölen, Sonnenblumenöl, Rapsöl oder Fischölen gewonnen sein.

Für Emulsionen mit sehr hohen, d.h. 90 Gew.-% übersteigenden Ölgehalten eignen sich vorzugsweise von $C_{12}$ - $C_{14}$-Fettsäuren abgeleitete Acylcyanamide.

...

Als Ölphase kommen beliebige nichtwäßrige, emulgierbare Verbindungen in Frage, in erster Linie jedoch aliphatische, cycloaliphatische, aromatische und alkylaromatische Kohlenwasserstoffe, ferner esterartige oder etherartige Fette und Wachse, Fettalkohole und Fettsäuren sowie deren Gemische. Der Anteil der nichtwäßrigen Phase in derartigen Emulsionen kann z.B. 70 - 95 Gew.-% betragen. Die Emulsionen zeichnen sich durch eine hohe Lagerstabilität auch bei erhöhter Temperatur aus und sind beliebig mit Wasser verdünnbar.

Die Emulsionen können zusätzliche Wirkstoffe enthalten, die sie für den jeweiligen Anwendungszweck geeignet machen. Hierzu zählen z.B. pharmakologisch bzw. therapeutisch wirksame Substanzen, Kosmetika, Farb- und Duftstoffe, Pigmente, Antioxydantien, Biocide, Herbicide, Korrosionsinhibitoren oder Oxidationsmittel sowie gegebenenfalls weitere Emulsionshilfsmittel, Verdickungsmittel bzw. die Konsistenz beeinflussende Zusätze.

Die Herstellung der Emulsionen erfodert keine besonderen Vorkehrungen und kann z.B. in der Weise erfolgen, daß man den Emulgator in der in Aussicht genommenen Wassermenge ggf. unter Erwärmen löst, die Ölphase hinzufügt und beide Phasen durchmischt. Bei Verwendung eines hochtourigen Mischers genügen im allgemeinen Mischzeiten von 1 - 2 Minuten zur Bildung homogener, stabiler Emulsionen. Ein stufenweises Einarbeiten der Ölphase ist bei Verwendung der erfindungsgemäß zu verwendenden Emulgatoren nicht erforderlich.

Emulsionen mit einem Anteil von 70 - 80 Gew.-% Ölphase sind im allgemeinen flüssig bis zähflüssig, solche mit höherem Ölgehalt meist von pastenartiger Konsistenz. Unter Scherbelastung zeigen sie ein thixotropes Verhalten, d.h. nach Beendigung der Scherbeeinflussung stellt sich nach kurzer Zeit wieder der ursprüngliche Viskositätszustand ein.

## Beispiele:

In den Beispielen wurden die folgenden Monoacylcyanamide in Form ihrer Natriumsalze als Emulgatoren eingesetzt.
(CA= Abkürzung für Cyanamid):

$E_1$ = Lauroyl-CA

$E_2$ = Myristoyl-CA

$E_3$ = Kokosfettsäure-CA

$E_4$ = Palmitoyl

$E_5$ = Stearoyl-CA

$E_6$ = Talgfettsäure-CA

Die ölige Phase bestand aus Mineralöl (Dieselöl), Paraffinöl, Testbenzin, Xylol, Ölsäuredecylester und 2-Octyldecanol. Zur Herstellung der Emulsionen wurde der Emulgator in destilliertem Wasser unter Erwärmen gelöst und anschließend die jeweilige Ölphase unter Rühren zugemischt. Die Rührvorrichtung wurde mit einer Tourenzahl von ca. 16000 - 17000 Umdrehungen pro Minute betrieben. Die Mischzeit (einschließlich der Zeit für die Zugabe der Ölphase) betrug 2 Minuten.

Die Stabilität der Emulsionen wurde während einer Standzeit von 3 bis 6 Wochen bei einer Lagertemperatur von 25 $^{\circ}$C, und nach 1 bis 3 Wochen bei einer Lagertemperatur von 40 $^{\circ}$C geprüft. Die Ergebnisse sind in den folgenden Tabelle aufgeführt.

Das Zeichen + gibt an, daß diese Emulsion keine Entmischungserscheinungen aufwies.

Einzelne Emulsionen wurden 52 Wochen bei 25 $^{\circ}$C gelagert, wonach ihre Viskosität bei 25 $^{\circ}$C mittels eines Rotationsviskosimeters nach BROOKFIELD bei Drehzahlen der Spindel von 6 bzw. 12 Umdrehungen pro Minute gemessen wurde.
Die Ergebnisse, die ebenfalls in der Tabelle aufgeführt sind, zeigen eine deutliche Thixotropie unter dem Einfluß der Scherwirkung der Meßspindel.

...

Tabelle I

| Beisp. | E Gew.-% | Ölphase Gew.-% | Stabilität 25° 6 W | 40° 3 W | Viskosität 6 U/Min mPas.s | 12 U/Min |
|--------|----------|----------------|--------------------|---------|----------------------------|----------|
| 1 | 0,5 % E1 | Mineralöl 75 % | + | + | - | - |
| 2 | 1,0 % E1 | | + | + | 10600 | 3320 |
| 3 | 1,0 % E2 | | + | + | 6200 | 2040 |
| 4 | 0,5 % E3 | | + | + | - | - |
| 5 | 1,0 % E3 | | + | + | 7200 | 2240 |
| 6 | 1,0 % E4 | | + | + | - | - |
| 7 | 1,0 % E5 | | + | + | - | - |
| 8 | 1,0 % E6 | | + | + | 4200 | 1300 |
| 9 | 1,0 % E1 | Mineralöl 90 % | + | + | - | - |
| 10 | 1,0 % E2 | | + | + | - | - |
| 11 | 1,0 % E4 | | + | + | - | - |
| 12 | 1,0 % E6 | | + | + | - | - |
| 13 | 1,0 % E2 | Mineralöl 95 % | + | + | - | - |
| 14 | 0,5 % E1 | | + | + | - | - |
| 15 | 1,0 % E1 | Paraffinöl 75 % | + | + | 4600 | 1580 |
| 16 | 1,0 % E2 | | + | + | - | - |
| 17 | 1,0 % E3 | | + | + | 10900 | 3200 |
| 18 | 1,0 % E6 | | + | + | 2800 | 930 |
| 19 | 1,0 % E1 | Paraffinöl 90 % | + | + | - | - |
| 20 | 1,0 % E6 | | + | + | - | - |
| 21 | 1,0 % E4 | Testbenzin 75% | + | + | - | - |
| 22 | 1,0 % E5 | | + | + | - | - |
| 23 | 1,0 % E1 | Testbenzin 90 % | + | + | - | - |
| 24 | 1,0 % E2 | | + | + | - | - |
| 25 | 1,0 % E4 | Xylol 75 % | + | + | - | - |
| 26 | 1,0 % E4 | Xylol 90 % | + | + | - | - |

. . .

**0115313**
HENKEL KGaA
ZR-FE/Patente

## Tabelle I (Fortsetzung)

| | | | | | | |
|---|---|---|---|---|---|---|
| 27 | 0,5 % E1 | 2-Octylde-canol 75 % | + | + | - | - |
| 28 | 1,0 % E1 | | + | + | 22000 | 5600 |
| 29 | 1,0 % E2 | | + | + | - | - |
| 30 | 1,0 % E3 | | + | + | 11000 | 3120 |
| 31 | 1,0 % E4 | | + | + | 4200 | 1360 |
| 32 | 1,0 % E6 | | + | + | 5000 | 1520 |
| 33 | 1,0 % E1 | 2-Octylde-canol 90 % | + | + | - | - |
| 34 | 1,0 % E6 | | + | + | - | - |
| 35 | 0,5 % E1 | Ölsäurede-cylester 75 % | + | + | - | - |
| 36 | 1,0 % E1 | | + | + | 4900 | 1480 |
| 37 | 1,0 % E2 | | + | + | - | - |
| 38 | 1,0 % E3 | | + | + | 13500 | 3800 |
| 39 | 1,0 % E4 | | + | + | 11200 | 3280 |
| 40 | 1,0 % E5 | | + | + | - | - |
| 41 | 1,0 % E6 | | + | + | 9200 | · 2700 |
| 42 | 1,0 % E2 | Ölsäurede-cylester 90 % | + | + | - | - |
| 43 | 1,0 % E6 | | + | + | - | - |

<u>P a t e n t a n s p r ü c h e</u>

1. Stabile Öl-in-Wasser-Emulsionen mit einem Gehalt an nichtwäßriger Phase von mehr als 60 Gew.-%, dadurch gekennzeichnet, daß der Emulgator aus wasserlöslichen Salzen von Monoacylcyanamiden mit 10 - 20 Kohlenstoffatomen in der Acylgruppe besteht.

2. Emulsionen nach Anspruch 1, dadurch gekennzeichnet, daß deren Gehalt an Emulgator 0,1 - 3 Gew.-% beträgt.

3. Emulsionen nach Anspruch 1 und 2, dadurch gekennzeichnet, daß die Monoacylcyanamide als Natriumsalz vorliegen.

4. Emulsionen nach Anspruch 1 - 3, dadurch gekennzeichnet, daß sich die Monoacylcyanamide von Fettsäuren mit 12 - 18 Kohlenstoffatomen ableiten.

5. Emulsionen nach Anspruch 1 - 4, dadurch gekennzeichnet, daß ihr Gehalt an nichtwäßriger Phase 70 - 95 Gew.-% beträgt.

6. Verwendung von wasserlöslichen Salzen von Monoacylcyanamiden, deren Acylrest 10 - 20 Kohlenstoffatome aufweist, als Emulgatoren für Öl-in-Wasser-Emulsionen mit einem Gehalt an nichtwäßriger Phase von mehr als 60 Gew.-%.

7. Verwendung von Salzen von Monoacylcyanamiden nach Anspruch 6 in Mengen von 0,1 - 3 Gew.-% der Emulsion.

8. Verwendung von Monoacylcyanamiden nach Anspruch 6 und 7 in Form der Natriumsalze.

9. Verwendung von Salzen von Monoacylcyanamiden nach Anspruch 6 - 8, deren Acylrest sich von linearen Fettsäuren mit 12 - 18 Kohlenstoff-atomen ableitet.

...

**0115313**
HENKEL KGaA
ZR-FE/Patente

10. Verwendung von Salzen von Monoacylcyanamiden nach Anspruch 6 - 9 zur Herstellung von Emulsionen mit einem Gehalt an nichtwäßriger Phase von 70 - 95 Gew.-%.